# EUROPEAN PATENT APPLICATION

(11) **EP 3 231 479 A1**
(43) Date of publication of application: **18.10.2017**
(21) Application number: 16164689.8
(22) Date of filing: 11.04.2016
(51) Int. Cl.: A61N 5/06, A61N 2/00, A61N 2/02, A61N 2/06

(54) **APPARATUS FOR PREVENTING OR REDUCING INFLAMMATION OF AT LEAST A PART OF A SKIN OF A BODY PART OF A HUMAN BODY**

(71) Applicant: Prosystem Health Products GmbH, 20097 Hamburg (DE)
(72) Inventor: Stettin, Jürgen, 20097 Hamburg (DE)
(74) Representative: RGTH

(57) **Abstract**

In order to effectively and safely prevent and reduce inflammation of at least a part of a skin of a body part as a reaction to the administration of a vascular endothelial growth factor inhibitor the present invention suggests an apparatus comprising at least one application unit, wherein the application unit has a receiving section for receiving a body part of a human body being disposed within an interior space of the application unit, wherein the application unit comprises at least one light emitter for applying light onto at least a part of the skin of the received body part within receiving section of the apparatus, wherein the application unit is configured to apply light onto at least a part of the skin of the received body part with a wavelength of between 400 nm to 500 nm, preferably a wavelength of between 430 nm to 480 nm, most preferred a wavelength of between 450 nm to 460 nm.

## Description

### Field of the invention

The invention relates to an apparatus for preventing or reducing inflammation of at least a part of a skin of a body part of a human body associated with an administration of a vascular endothelial growth factor inhibitor by application of light. Furthermore, the invention is concerned with a method for applying light onto at least a part of a skin of a body part of a human body as well as a computer software product.

### Description of Related Art

Apparatuses to apply light onto a part of a skin of a human body part are known in prior art. EP 0 228 537 A2, e.g., discloses a treatment device which is capable of applying infrared light and magnetic field onto the skin of body parts.

However, none of the apparatuses of prior arts are configured such as to effectively and at the same time safely prevent and reduce inflammation of skin parts inflamed as a reaction to the administration of a vascular endothelial growth factor inhibitor.

In prior art most solutions to prevent or reduce an inflammation of skin associated with an administration of a vascular endothelial growth factor inhibitor are non pharmacological. For example unnecessary pressure or friction of the skin should be avoided as well as activities which broaden the blood vessels, such as taking a hot shower or exposing the skin to sunlight. Those measures, however, limit the life quality of the affected persons severely since most activities require at least a minimum movement and thus also friction and pressure of the hands or the feet, such as e.g. taking a walk.

Furthermore, it is known to apply ointments onto the affected skin parts to reduce symptoms related to the inflammation. However, the application of the ointments onto already inflamed skin parts can be very painful, while at the same time known ointments do not prevent the development of an inflammation.

### Summary of the Invention

It is an objective of the present invention to design an apparatus for preventing or reducing inflammation of at least a part of a skin of a body part of a human body associated with an administration of a vascular endothelial growth factor inhibitor by means of applying light. In this regard, the apparatus should ensure an effective prevention or reduction of the inflammation, while at the same time sufficient safety to the person onto whose skin light is applied is provided.

The above objective is solved by providing an apparatus for preventing or reducing inflammation of at least a part of a skin of a body part of a human body associated with an administration of a vascular endothelial growth factor inhibitor by application of light wherein the apparatus comprises at least one application unit, which has a receiving section for receiving a body part of a human body. This receiving section is disposed within an interior space of the application unit. The application unit comprises at least one light emitter for applying light onto at least a part of the skin of the received body part within receiving section of the apparatus. The application unit is configured to apply light onto at least a part of the skin of the received body part, in particular within the receiving section, with a wavelength of between 400 nm to 500 nm, preferably a wavelength of between 430 nm to 480 nm, most preferred a wavelength of between 450 nm to 460 nm.

It has been found that blue light acts anti-inflammatory and reduces and prevents the proliferation of keratinocytes being a type of cell which is predominant in the epidermis of skin. The apparatus is preferably an irradiation device, being able to expose a part of a skin of a received body part to light with the above named wavelengths. The applied light preferably comprises a spectrum of wavelengths, advantageously a small spectrum of wavelength, which lies within the above named ranges. In particular, the application unit is configured to apply only light of the above ranges, and no other wavelengths, onto the skin of the received body part.

This exposure occurs within the application unit due to the receiving section being disposed in an interior space of the application unit. The receiving section is designed such that the body part onto whose skin light is to be applied can be inserted into it. In this way, sensitive other body parts, such as the eyes, of the person onto whose skin light is applied are protected from the applied light. The application unit, in particular its receiving section, is configured such that applied light exiting to an outside of the application unit is prevented or at least reduced and at the same time a best possible ergonomic positioning of the body part can be achieved. Due to the fact that the light is applied within the receiving section of the application unit a predetermined and effective application of light is ensured since there is not uncertainty about any otherwise unknown parameters, such as e.g. the distance of the body part to the application unit. Furthermore, the application unit is configured such that a homogeneous application of light is assured within the receiving section.

In this way, and especially by using the above named wavelengths, an effective prevention and reduction of a skin inflammation can be achieved, while at the same time it is prevented that applied light exits to an outside of the application unit as a safety precaution for the person onto whose skin light is applied.

The body part is preferably a hand or a foot of a human body, i.e. a person. The skin part is in particular the palm of a hand or the sole of a foot. The apparatus is in particular configured to prevent and reduce inflammation and/or any other symptoms such as e.g. swelling, itching, numbness, associated with the hand-foot-syndrome. The hand-foot-syndrome is a common skin toxicity of the administration of a vascular endothelial growth factor inhibitor which is used in chemotherapy. The hand-foot syndrome, also called palmar-plantar erythrodysesthesia, is characterized by reddening, swelling, numbness and desquamation (skin sloughing or peeling) of the hands, especially the palms, and feet, in particular the soles. The receiving section is in particular formed such that at least one hand and/or one foot, preferably either both hands or both feet, can be placed in it at the same time.

Most preferably, the apparatus is configured such that the pressure to affected skin parts is kept to a minimum. In particular, the receiving section comprises a supporting surface onto which the body parts received within the receiving section can be placed. The application unit does in particular not comprise any part applying an additional pressure onto the affected skin parts apart from gravity, such as for example an inflatable part of the application unit.

The at least one light emitter is preferably configured to emit light with a wavelength of 400 nm to 500 nm, preferably a wavelength of 430 nm to 480 nm, most preferred a wavelength of 450 nm to 460 nm. Alternatively, the light emitter could emit white light, wherein the application unit further comprises filters, such as e.g. bandpass filters, in order to let only light of the above named wavelengths pass into the receiving section. The application unit is provided with a battery or is connected to the power supply.

Preferably, the application unit comprises a housing defining the receiving section of the application unit. In particular, the housing of the application unit encloses the interior space of the application unit for the most part, especially entirely except for at least one, preferably two, openings in the housing for introducing body parts into the interior space and thus the receiving section of the application unit. Especially, the housing encloses the interior space in circumferential direction, while the at least one opening is disposed at a front side of the application unit. In the case of two openings these are preferably disposed opposite of each other.

A first section of the housing has at least one translucent layer for letting light emitted by the at least one light emitter pass from the at least one light emitter into the receiving section of the application unit. In particular, the translucent layer forms at least the radial innermost layer of the first section. Furthermore, the translucent layer is configured as a supporting surface onto which the body part received within the receiving section can be placed.

The housing preferably has four different sections, a first section and an opposite second section as well as a third section and an opposite fourth section. Especially, the first section is disposed at the bottom of the application unit, which is placed e.g. on the floor or on a table, whereas the second section is disposed at the top of the application unit and the third and the fourth sections are disposed at the sides of the application unit. Each of the first section and the second section is arranged adjoining the third section and the fourth section, especially in a corner area of the housing of the application unit and particularly at an angle of about 90 ° so that the application unit has a rectangular shape. The first to fourth sections enclose the interior space of the application unit in circumferential direction. In addition, there can be a fifth section closing the interior space from at least one front side.

The translucent layer is preferably disposed in thickness direction of the first section before the at least one light emitter so that light can pass from the light emitter to an interior space of the application unit, especially the receiving section. The "thickness direction" corresponds to a transverse direction or cross direction of a section of the housing.

Preferably, the translucent layer comprises glass, preferably acrylic glass, i.e. poly(methyl methacrylate). In particular, the emitted light has a spectrum of emitted wavelengths wherein the translucent layer is configured to let only the spectrum of the emitted wavelengths pass. This has the advantage over normal glass that as little light as possible from the desired wavelengths is attenuated by the glass. Alternatively, the translucent layer is formed by a jelly-like material, preferably a gel cushion. Using a jelly-like material has the advantage that it is flexible so that it can adapt to e.g. the shape of the received body part or any movement by it.

Advantageously, the application unit comprises a plurality of light emitters, wherein the light emitters are disposed within the first section of the housing. In another preferred embodiment, light emitters are only disposed in the first section, i.e. no light emitters are disposed within the other sections of the housing.

Alternatively, light emitters are, preferably only, disposed in the first section as well as the third and the fourth section, in particular in parts of the third section and the fourth section adjoining the first section so that no light emitters are disposed in the second section. Due to the arrangement of the light emitters not only the skin which faces the first section is exposed to the emitted light, but also side parts of the skin being opposed to the third or the fourth section can be exposed to light. In this case, the second and the third section also comprise a translucent layer, at least in the parts in which light emitters are disposed.

Generally, all sections of the housing within which light emitters are disposed preferably comprise a translucent layer so that the light emitted from the light emitters can pass from the light emitters being disposed inside of the section of the housing to the receiving section of the application unit.

Furthermore, light emitters can be disposed within the first and the second section of the housing, but not in the third and fourth section. In another embodiment, light emitters are disposed within the housing along its entire circumferential direction.

The light emitters are particularly formed by light emitting dioses, LEDs, or by light conductors. The light emitters are preferably disposed within an interior space within the respective section of the housing wherein at least part of the section of the housing defining the interior space has at least one reflecting layer for reflecting light emitted from the at least one light emitter.

A section of the housing comprising light emitters, e.g. the first section, preferably comprises three different layers in its thickness direction wherein the first layer is the outer most layer of the section, the second layer is formed by an interior space of the section and the third layer by the translucent layer. In the interior space and on top of the first layer light emitters are disposed.

The interior space within the section is preferably enclosed by the section of the housing. The interior space of the section is preferably limited by the surface areas of the third layer and the first layer of the section facing each other as well as lateral sidewalls also facing each other. In particular, the lateral sidewalls are coated with a reflecting material so that light emitted from the light emitters is reflected at the lateral sidewalls and as much light as possible is emitted into the receiving section of the application unit.

In addition, the application unit can be configured to generate a magnetic field and apply the magnetic field onto at least a part of the skin of the received body part. For this purpose, the application unit comprises at least one magnet for producing a magnetic field. In particular, the light and the magnetic field can be applied simultaneously onto a part of the skin of a received body part. In order to generate a magnetic field, the application unit preferably comprises a plurality of magnets. Those magnets can be disposed only within the first section, the second section, the third section or the fourth section of the housing. They are advantageously disposed within the first section and the second section or the third section and the fourth section. Especially, the magnets are arranged in such a way that a homogeneous magnetic field within the receiving section results. Instead or addition to magnets, the application unit can comprise windings which form a coil generating the magnetic field. Those windings are preferably disposed within the housing and in particular arranged in such a way that they enclose the receiving section. In a further preferred embodiment, the application unit is configured to generate, either by means of magnets and/or windings, an oscillating magnetic field. The frequency of the oscillating magnetic field lies preferably between 25 Hz to 50 Hz, wherein the magnetic flux density is advantageously between 10 mikro Tesla and 20 mikro Tesla and the intensity is between 400 mW/*cm*² to 800 mW/*cm*², preferably between 500 mW/*cm*² and 700 mW/*cm*² or most preferred between 550 mW/*cm*² and 650 mW/*cm*².

Preferably, the application unit has the approximate shape of a cuff, a sock, a sack, a glove or a shoe. This means, that the outer and/or the inner shape of the application unit is shaped as a cuff, a sock, a sack, a glove or a shoe. Especially, the outer and/or the inner shape of the application unit are adapted to the shape of a hand or a foot. In particular, the housing of the application unit has a curved shape in cross section, preferably the cross section has a round or oval shape. The application unit can have a separate protrusion for each finger of a hand or for each toe of a foot. When having the form of a shoe, the first section of the application unit can be formed as a sole of the shoe comprising the light emitters.

Particularly preferred, the housing of the application unit is at least partly, preferably substantially, made of an elastic material for allowing a change of shape of the housing. Especially, the housing is configured such out of elastic material that the application unit allows an adaption to the shape of the body part and/or to a movement of the received body part, especially a hand or the fingers of a hand or a foot or the toes of a foot, within the application unit. Due to the elasticity of the material, it is further achieved that the body part can cozily and in a relaxed state be rested within the application. This means that the possibly already inflamed skin is affected as little as possible. In particular, no additional pressure is applied onto the skin. For this purpose, the application unit preferably comprises a textile, opaque material for forming the housing. Furthermore the application unit particularly comprises a jelly-like material for forming a translucent layer as well as light conductors, especially fiber optics, for forming the light emitters. The light conductors forming the light emitters extend in particular approximately along the longitudinal and/or the cross direction of the application unit, preferably at a constant distance to each other.

The apparatus furthermore comprises at least one control unit for controlling the application unit. The control unit is preferably configured to control the emittance of light and the generation of a magnetic field independently from each other. Furthermore, the control system can be configured to determine a radiant exposure of the applied light onto the skin part of the received body part, compare it to a predetermined maximum value of radiant exposure and stop the operation of the apparatus onto the skin part as soon as the predetermined maximum value of radiant exposure has been achieved. As a consequence, the control system has a safety precautionary measure in order to avoid too much exposure to a skin part which can cause various damages to the skin. The predetermined value of radiant exposure per day is set to be between 50 J/*cm*² to 110 J/*cm*², preferably between 65 J/*cm*² and 105 J/*cm*², most preferred between 80 J/*cm*² and 100 J/*cm*².

Moreover, the apparatus can comprise a plurality of application units. Preferably, the application unit has two application units, e.g. one for each hand and/or each foot. The apparatus can comprise a plurality of control units, wherein one control unit is assigned to each application unit. Preferably, the control unit of each application unit is disposed in the respective application unit. In addition, the apparatus can comprise a main control unit controlling the other control units which may or may not be disposed in one of the application units. Alternatively, the apparatus comprises one common control unit for all application units which is configured to control all of the application units. Advantageously, the control units of the application units are configured to communicate with each other and/or with a central control unit, by wire or by wireless communication, e.g. radio communication, Bluetooth or Wi-Fi.

In another aspect, the invention relates to a method for applying light onto at least a part of a skin of a body part of a human body, wherein light is applied to a part of a skin of a body part of a human body. The light applied to the part of the skin has a wavelength of 400 nm to 500 nm, preferably a wavelength of 430 nm to 480 nm, most preferred a wavelength of 450 nm to 460 nm and wherein the maximum daily radiant exposure of the applied light onto the part of the skin is between 50 J/*cm*² to 110 J/*c*², preferably between 65 J/*cm*² and 105 J/*cm*², most preferred between 80 J/*cm*² and 100 J/*cm*². Furthermore preferred, the method includes the step of applying the light onto the skin part for a period of time of about 30 minutes per day.

Preferably, the method further comprises the steps of determining a radiant exposure of the applied light onto the skin part, comparing it to a predetermined maximum value of radiant exposure and stopping an emittance of light as soon as the predetermined maximum value of radiant exposure has been achieved. The predetermined value of radiant exposure per day is set to be between 50 J/*cm*² to 110 J/*cm*², preferably between 65 J/*cm*² and 105 J/*cm*², most preferred between 80 J/*cm*² and 100J/*cm*².

In addition, the method can comprise applying a magnetic field onto the part of the skin of a human body. Preferably, an oscillating magnetic field is applied, wherein the frequency of the oscillating magnetic field lies preferably between 25 Hz to 50 Hz, the magnetic flux density is advantageously between 10 and 20 mikro Tesla and the intensity is between 400 mW/*cm*² to 800 mW/*cm*², preferably between 500 mW/*cm*² and 700 mW/*cm*² or most preferred between 550 mW/*cm*² and 650 mW/*cm*².

Preferably, the method comprises identifying a status of the state of the part of the skin onto which light is to be applied which in particular comprises making observation of the part of the skin, comparing the observations with observations of a database and assigning a status to the skin. The status can e.g. be a degree of inflammation. For example, the inflammation associated with hand-foot syndrome is characterized in CTC (common toxicity criteria) grades 1 to 4. According to the status of the skin the method comprises choosing from a plurality of application programs a most applicable application program. The application programs preferably differ in the intensity of the magnetic field which is applied. The more severe the inflammation is, the higher the applied magnetic flux density is, while at the same time the intensity of the applied light is preferably constant.

In a further aspect of the invention, a computer software product including a medium readable by a processor is claimed wherein on the medium a program for applying light on at least a part of the skin of a body part of a human body for preventing or reducing inflammation associated with an administration of a vascular endothelial growth factor inhibitor is stored which enables a computer, after being loaded into a memory of the computer, to apply light onto a part of a skin of a body part of a human body wherein the light applied to the part of the skin has a wavelength of 400 nm to 500 nm, preferably a wavelength of 430 nm to 480 nm, most preferred a wavelength of 450 nm to 460 nm and wherein the maximum daily radiant exposure of the applied light onto the skin part is between 50 J/*cm*² to 110 J/*cm*², preferably between 65 J/*cm*² and 105 J/*cm*²*,* most preferred between 80 J/*cm*² and 100 J/*cm*²*.* In particular, the program enables a computer to conduct a method as described above.

### Brief Description of the Drawings

The invention will be described below with reference to the following figures which show in schematic representation:
- Figure 1: a perspective view on an apparatus for preventing or reducing inflammation of at least a part of a skin of a body part of a human body associated with the administration of a vascular endothelial growth factor inhibitor by application of light;
- Figure 2: a cross sectional view of the apparatus of figure 1;
- Figure 3: a cross sectional view of another apparatus for preventing or reducing inflammation of at least a part of a skin of a body part of a human body associated with the administration of a vascular endothelial growth factor inhibitor by application of light;
- Figure 4: a perspective view of another apparatus for preventing or reducing inflammation of at least a part of a skin of a body part of a human body associated with the administration of a vascular endothelial growth factor inhibitor by application of light;
- Figure 5: a cross sectional view of the apparatus of figure 4;
- Figure 6: a perspective view of another apparatus for preventing or reducing inflammation of at least a part of a skin of a body part of a human body associated with the administration of a vascular endothelial growth factor inhibitor by application of light;
- Figure 7: a perspective view of another apparatus for preventing or reducing inflammation of at least a part of a skin of a body part of a human body associated with the administration of a vascular endothelial growth factor inhibitor by application of light;
- Figure 8: a perspective view of another apparatus for preventing or reducing inflammation of at least a part of a skin of a body part of a human body associated with the administration of a vascular endothelial growth factor inhibitor by application of light; and
- Figure 9: a cross sectional view of the apparatus of figure 8.

### Detailed Description of the Drawings

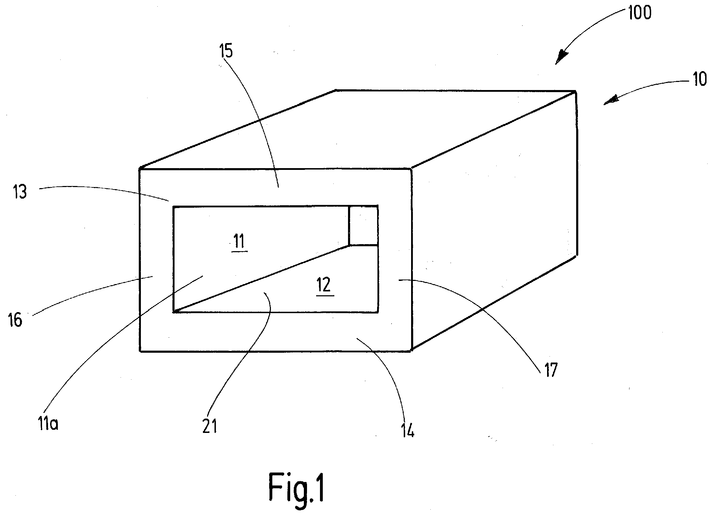

Figure 1 shows a perspective view on an apparatus (100) for preventing or reducing inflammation of at least a part of a skin of a body part of a human body associated with the administration of a vascular endothelial growth factor inhibitor by application of light.

The apparatus (100) comprises one application unit (10). The application unit (10) has a rectangular shape and comprises a receiving section (11) for receiving a body part of a human body which is disposed in an interior space (12) of the application unit (10) and also has a rectangular shape. For introducing a body part into the receiving section (11) the application unit (10) has an opening (11a). The receiving section (11) is in particular formed such that either both hands or both feet can be placed in the receiving section (11) at the same time.

The application unit (10) further comprises a housing (13) which defines the receiving section (11). The housing (13) has four different sections, a first section (14) and an opposite second section (15) as well as a third section (16) and an opposite fourth section (17). The first section (14) is disposed at the bottom of the application unit (10), whereas in the second section (15) is disposed at the top of the application unit (10). The third and the fourth sections (15, 16) are disposed at the sides of the application unit (10). Both the first section (14) and the second section (15) are arranged adjoining the third section (16) and the fourth section (17), especially in a corner area of the housing (13) of the application unit (100) and particularly at an angle of about 90 °.

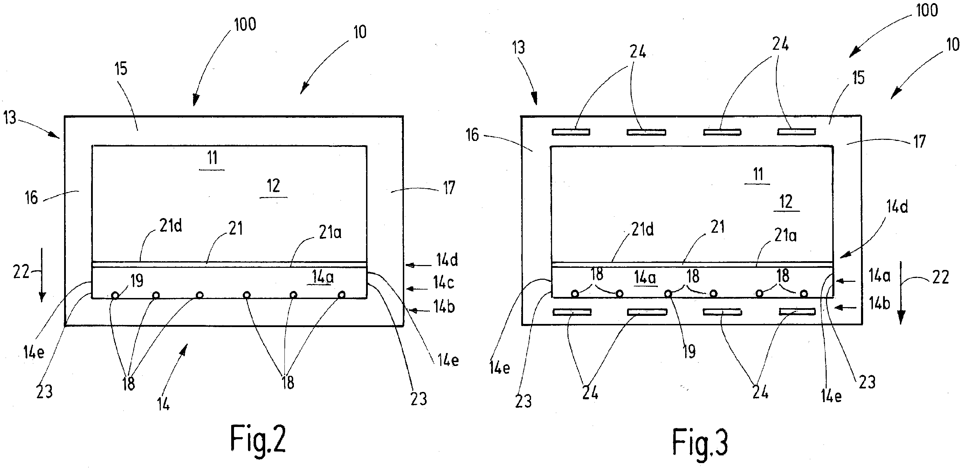

In figure 2 a cross sectional view of the apparatus (100) of figure 1 is shown. The first section (14) of the housing (13) comprises three different layers in its thickness direction (22). In figure 1 the thickness direction (22) of the first section (14) is shown which is disposed at an angle of 90° to the first section (14). The first layer (14b) forms the outer most layer of the first section (14). The second layer (14c) is formed by an interior space (14a) of the first section (14). In the interior space (14a) and on top of the first layer (14b) light emitters (18) in the form of LEDs (19) are disposed. The third layer (14d) is formed by a translucent layer (21) so that light emitted by the light emitters (14) can exit into the receiving section (11) of the application unit (10).

The translucent layer (21) is formed by an acrylic glass plate (21a) which is disposed parallel to the first layer (14b) of the first section (14). The translucent layer (21), more specifically the surface of the acrylic glass plate (21a) facing the receiving section (11), is formed as a supporting surface (21d) onto which the body part to be inserted in the receiving section (11) can be placed. The interior space (14a) of the first section (14) is limited by the surface areas of the third layer (14d) and the first layer (14b) facing each other as well as lateral sidewalls (14e) also facing each other. The lateral sidewalls (14e) are coated with a reflecting material (23) so that light emitted from the light emitters (18) is reflected at the lateral sidewalls (14e) and as much light as possible is emitted into the receiving section (11) of the application unit (10).

Figure 3 shows a cross sectional view of another apparatus (100) for preventing or reducing inflammation of at least a part of a skin of a body part of a human body associated with the administration of a vascular endothelial growth factor inhibitor by application of light. The application unit (10) of the apparatus (100) is configured in the same way as the application unit (10) of figure 1 except for the fact that the application unit (10) of figure 2 comprises magnets (24) in order to generate a magnetic field within the receiving section (11) of the application unit (10). The magnets (24) are disposed within the first layer (14b) of the first section (14) as well as the second section (15) of the housing (13). By means of the magnets (24) a homogeneous magnetic field within the receiving section (11) of the application unit (10) is generated.

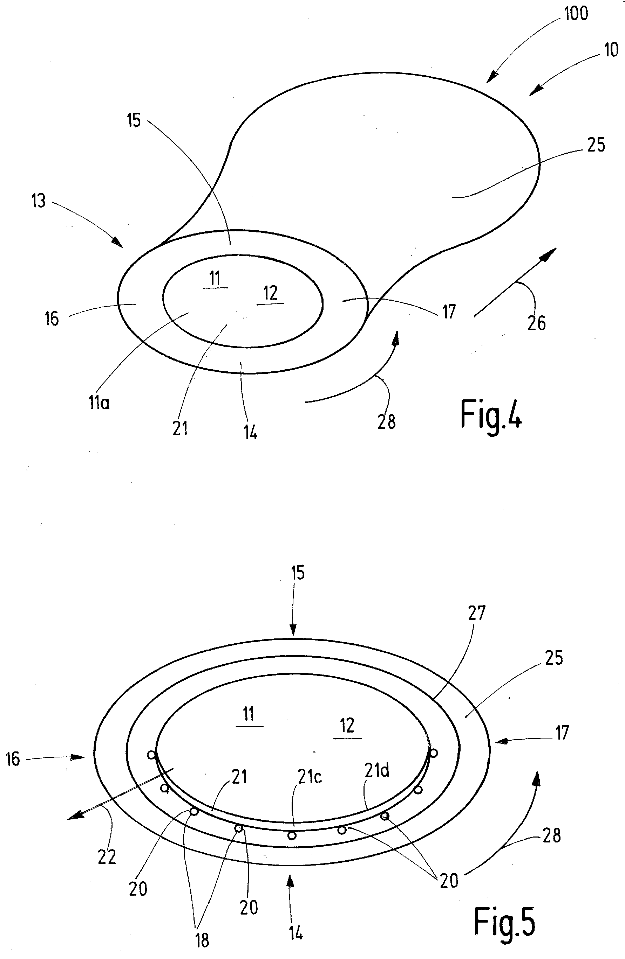

In Figure 4 a perspective view of another apparatus (100) for preventing or reducing inflammation of at least a part of a skin of a body part of a human body associated with the administration of a vascular endothelial growth factor inhibitor by application of light is shown. The apparatus (100) comprises one application unit (10) which has the shape of a sack. The application unit (10) has a receiving section (11) in its interior space (12) which can be accessed via an opening (11a). The housing (13) of the application unit (10) comprises an elastic material (25) which allows the application unit (10) to adapt to the shape of the body part to be inserted. Furthermore, the elasticity allows a movement of the received body part within the application unit (10) as well as comfortable position within it.

Figure 5 shows a cross sectional view of the apparatus (100) of figure 4. Due to the sack-like form the application unit (10) has an oval cross section so that the thickness direction (22) substantially coincides with the radial direction of the application unit (10). The application unit (10) comprises a receiving section (11) for receiving a body part in the interior (12) of the application unit (10). The receiving section (12) is surrounded by the housing (13) of the application unit (10) which can roughly be divided into four sections (14, 15, 16, 17), a first section (14) being opposite of a second section (15) and a third section (16) being opposite of a fourth section (17).

As light emitters (14) the application unit (10) of figures 4 and 5 comprises light conductors (20) which are disposed within the first section (14) as well as an adjoining part of the third section (16) and the fourth section (17). The light conductors (20) extend approximately along the longitudinal direction (26) of the application unit (10), preferably at a constant distance to each other. Along substantially their entire length the light conductors (20) can emit light.

As the translucent layer (21) the application unit (10) comprises a jelly-like material (21b), namely a gel cushion (21c), which contributes to the elasticity of the application unit (10). The innermost surface of the translucent layer (21) is formed as a supporting surface (21d) for supporting the received body part.

The application unit (10) further comprises windings (27) for generating a magnetic field within the receiving section (11). Part of one of these windings (27) is shown in figure 5. The windings (27) surrounds the receiving section (11) in the circumferential direction (28) of the application unit (10).

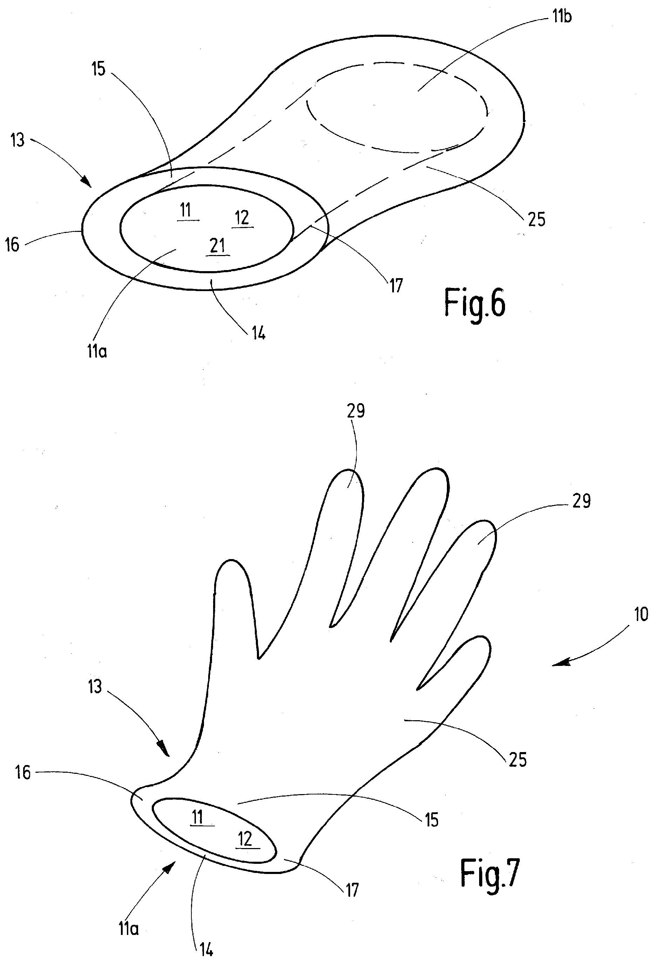

Figure 6 shows a perspective view of another apparatus (100) for preventing or reducing inflammation of at least a part of a skin of a body part of a human body associated with the administration of a vascular endothelial growth factor inhibitor by application of light wherein its application unit (10) is formed as a cuff. The application unit (10) of figure 6 is formed in the same way as the application unit (10) of figures 4 and 5 except for the fact that the application unit (10) has two openings (11a, 11b) to its receiving section (11) due to its cuff-like shape.

In figure 7 a perspective view of another apparatus (100) for preventing or reducing inflammation of at least a part of a skin of a body part of a human body associated with the administration of a vascular endothelial growth factor inhibitor by application of light is shown. The apparatus (100) comprises two application units (10) wherein each is formed as a glove and from which one is shown in figure 7.

The configuration of the application unit (10) of figure 7 corresponds basically to the configuration of the application unit (10) of figures 4 and 5 except for the following: Owed to its glove-like shape the application unit (10) has finger-formed protrusions (29) for each finger of a hand to be inserted into the application unit (10). The light conductors (20) are configured such that at least one light conductor (20) extends along substantially the entire length of each finger-like protrusion (29). The apparatus (100) has a common control system for controlling the two application units (10). The common control system is configured to communicate with each of the application units (10).

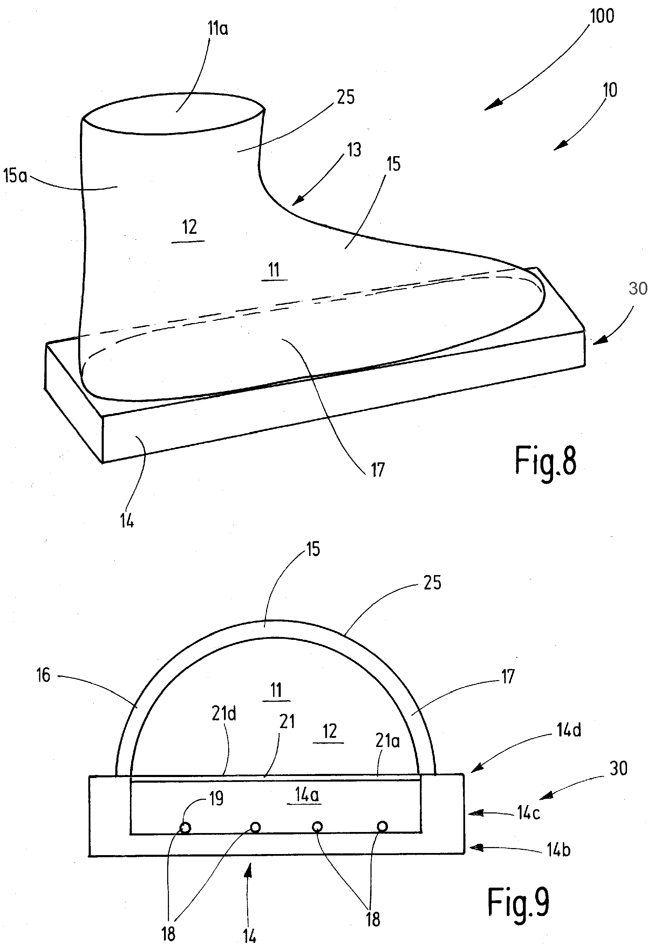

Figure 8 shows a perspective view of another apparatus (100) for preventing or reducing inflammation of at least a part of a skin of a body part of a human body associated with the administration of a vascular endothelial growth factor inhibitor by application of light. The application unit (10) is formed as a shoe. The receiving section (11) is formed by the interior space (12) of the shoe-shaped application unit (10).

Furthermore, the application unit (10) comprises a housing (13). The housing (13) is formed such that an entire foot inserted into the receiving space (11) can be surrounded. The housing (13) of the application unit (10) can roughly be divided into four sections (14, 15, 16, 17), a first section (14) being opposite of a second section (15) and a third section (16) being opposite of a fourth section (17). The first section (14) of the housing (13) of the application (10) is formed by the sole (30) of the shoe-shaped application unit (10). The second section (15) of the housing (14) has a tubular part (15a) into which a foot can be inserted. For this purpose, the second section (15), in particular the tubular part (15a) has an opening (11a).

In figure 9 a cross sectional view of the apparatus (100) of figure 8 is shown. Owed to the shoe like form, the cross section is formed oval. The first section (14) of the housing (13) is the only section comprising light emitters (18). The second, third and fourth sections (15, 16, 17) of the housing comprises elastic material (25) allowing the application unit (10) to adapt to the shape of the body part, i.e. a foot, to be inserted.

The first section (14) of the housing (13) comprises three different layers in its thickness direction (22), the first layer (14b) forming the outer most layer of the first section (14). The second layer (14c) is formed by an interior space (14a) of the first section (14). In the interior space (14a) and on top of the first layer (14b) light emitters (18) in the form of LEDs (19) are disposed. The third layer (14d) forms the innermost part of the first section (14). The innermost layer facing the receiving section (11) is formed by a translucent layer (21) so that light emitted by the light emitters (18) can exit into the receiving section (11) of the application unit (10). The translucent layer (21) is formed by an acrylic glass plate (21a).

### List of reference signs

- 100: apparatus

- 10: application unit
- 11: receiving section
- 11a, 11b: openings of receiving section
- 12: interior space of the application unit
- 13: housing
- 14: first section of housing
- 14a: interior space of first section
- 14b: first layer of first section
- 14c: second layer of first section
- 14d: third layer of first section
- 14e: lateral sidewalls of first section15 second section of housing
- 15a: tubular part
- 16: third section of housing
- 17: fourth section of housing
- 18: light emitters
- 19: LED

- 20: light conductor
- 21: translucent layer
- 21a: acrylic glass plate
- 21b: jelly-like material
- 21c: gel cushion
- 21d: supporting surface
- 22: thickness direction
- 23: reflective layer
- 24: magnets
- 25: elastic material
- 26: longitudinal direction
- 27: winding
- 28: circumferential direction
- 29: Protrusion
- 30: sole

## Claims

1. An apparatus (100) for preventing or reducing inflammation of at least a part of a skin of a body part of a human body associated with an administration of a vascular endothelial growth factor inhibitor by application of light,
**characterized in that**
the apparatus (100) comprises at least one application unit (10),
wherein the application unit (10) has a receiving section (11) for receiving a body part of a human body,
wherein the receiving section (11) is disposed within an interior space (12) of the application unit (10),
wherein the application unit (10) comprises at least one light emitter (18) for applying light onto at least a part of the skin of the received body part within the receiving section (11) of the apparatus (100),
wherein the application unit (10) is configured to apply light onto at least a part of the skin of the received body part with a wavelength of between 400 nm to 500 nm, preferably a wavelength of between 430 nm to 480 nm, most preferred a wavelength of between 450 nm to 460 nm.

2. The apparatus (100) according to claim 1,
**characterized in that**
the application unit (10) comprises a housing (13) defining the receiving section (11) of the application unit (10),
wherein a first section (14) of the housing (13) has at least one translucent layer (21) for letting light emitted by the at least one light emitter (18) pass from the at least one light emitter (18) into the receiving section (11) of the application unit (10).

3. An apparatus (100) according to claim 1 or 2,
**characterized in that**
the application unit (10) comprises a plurality of light emitters (18),
wherein the light emitters (18) are disposed within the first section (14) of the housing (13).

4. An apparatus (100) according to any of the preceding claims,
**characterized in that**
the application unit (10) is configured to generate a magnetic field and apply the magnetic field onto at least a part of the skin of the received body part.

5. An apparatus (100) according to any of the preceding claims,
**characterized in that**
the application unit (10) has the approximate shape of a cuff, a sock, a sack, a glove or a shoe.

6. An apparatus (100) according to any of claims 2 to 5,
**characterized in that**
the housing (13) of the application unit (10) is at least partly made of an elastic material for allowing a change of shape of the housing (13).

7. An apparatus (100) according to any of the preceding claims,
**characterized in that**
the apparatus (100) comprises at least one control unit for controlling the application unit (10).

8. A method for applying light onto at least a part of a skin of a body part of a human body, in particular for preventing or reducing inflammation associated with an administration of a vascular endothelial growth factor inhibitor, wherein light is applied to a part of a skin of a body part of a human body,
**characterized in that**
the light applied has a wavelength of 400 nm to 500 nm, preferably a wavelength of 430 nm to 480 nm, most preferred a wavelength of 450 nm to 460 nm,
wherein the maximum daily radiant exposure of the applied light onto the part of the skin is between 50 J/*cm*² to 110 J/*cm*², preferably between 65 J/*cm*² and 105 J/*cm*², most preferred between 80 J/*cm*² and 100 J/*cm*².

9. The method according to claim 8,
**characterized in that**
the method comprises the steps of determining a radiant exposure of the applied light onto the part of the skin, comparing it to a predetermined maximum value of radiant exposure and stopping an emittance of light as soon as the predetermined maximum value of radiant exposure has been achieved.

10. The method according to any of claims 8 or 9,
**characterized in that**
the method comprises applying a magnetic field onto the part of the skin.

11. The method according to any of claims 8 to 10,
**characterized in that**
the method comprises identifying a status of the state of the part of the skin onto which light is to be applied.

12. The method according to any of claim 11,
**characterized in that**
the step of identifying the status of the part of the skin comprises making observation of the part of the skin, comparing the observations with observations of a database and assigning a status to the skin.

13. The method according to any of claims 11 or 12,
**characterized in that**
the method comprises choosing from a plurality of application programs a most applicable application program regarding the status of the part of the skin.

14. A computer software product including a medium readable by a processor, the medium having stored thereon a program for applying light on at least a part of the skin of a body part of a human body for preventing or reducing inflammation associated with an administration of a vascular endothelial growth factor inhibitor, said program enabling a computer, after being loaded into a memory of the computer, to apply light onto a part of a skin of a body part of a human body wherein the light applied to the part of the skin has a wavelength of 400 nm to 500 nm, preferably a wavelength of 430 nm to 480 nm, most preferred a wavelength of 450 nm to 460 nm and wherein the maximum daily radiant exposure of the applied light onto the part of the skin is between 50 J/*cm*² to 110 J/*cm*², preferably between 65 J/*cm*² and 105 J/*cm*², most preferred between 80 J/*cm*² and 100 J/*cm*².
